(19) Europäisches Patentamt / European Patent Office / Office européen des brevets

(11) **EP 3 450 555 B1**

(12) **EUROPEAN PATENT SPECIFICATION**

(45) Date of publication and mention
of the grant of the patent:
**18.11.2020  Bulletin 2020/47**

(51) Int Cl.:
***C12N 15/09*** (2006.01)     ***C12Q 1/68*** (2018.01)

(21) Application number: **17789224.7**

(86) International application number:
**PCT/JP2017/014517**

(22) Date of filing: **07.04.2017**

(87) International publication number:
**WO 2017/187937 (02.11.2017 Gazette 2017/44)**

(54) **METHOD FOR DETERMINING TOLERANCE OF CANCER CELL TO EPIDERMAL GROWTH FACTOR RECEPTOR INHIBITOR**

VERFAHREN ZUR BESTIMMUNG DER TOLERANZ VON KREBSZELLEN GEGENÜBER DEM EPIDERMALEN WACHSTUMSFAKTORREZEPTORINHIBITOR

PROCÉDÉ DE DÉTERMINATION DE LA TOLÉRANCE D'UNE CELLULE CANCÉREUSE À UN INHIBITEUR DU RÉCEPTEUR DU FACTEUR DE CROISSANCE ÉPIDERMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2016  JP 2016091801**

(43) Date of publication of application:
**06.03.2019  Bulletin 2019/10**

(73) Proprietors:
• **Denka Company Limited**
  **Tokyo 103-8338 (JP)**
• **Niigata University**
  **Niigata-shi**
  **Niigata 950-2181 (JP)**

(72) Inventors:
• **IZUTSU, Hiroshi**
  **Machida-city**
  **Tokyo 194-8560 (JP)**
• **KODAMA, Keisuke**
  **Machida-city**
  **Tokyo 194-8560 (JP)**
• **NAKADA, Mitsutaka**
  **Machida-city**
  **Tokyo 194-8560 (JP)**
• **WAKAI, Toshifumi**
  **Niigata-shi**
  **Niigata 951-8510 (JP)**
• **KAMEYAMA, Hitoshi**
  **Niigata-shi**
  **Niigata 951-8510 (JP)**
• **NAGAHASHI, Masayuki**
  **Niigata-shi**
  **Niigata 951-8510 (JP)**
• **SHIMADA, Yoshifumi**
  **Niigata-shi**
  **Niigata 951-8510 (JP)**
• **ICHIKAWA, Hiroshi**
  **Niigata-shi**
  **Niigata 951-8510 (JP)**

(74) Representative: **Enomoto, Kéi et al**
**Maucher Jenkins**
**26 Caxton Street**
**London SW1H 0RJ (GB)**

(56) References cited:
**JP-A- 2014 533 960**

• **ZIAI JAMES ET AL: "BRAF mutation testing in clinical practice", EXPERT REVIEW OF MOLECULAR DIAGNOSTICS, EXPERT REVIEWS LTD, GB, vol. 12, no. 2, 1 March 2012 (2012-03-01), pages 127-138, XP008176589, ISSN: 1744-8352, DOI: 10.1586/ERM.12.1**
• **WENDY DE ROOCK ET AL: "KRAS, BRAF, PIK3CA, and PTEN mutations: implications for targeted therapies in metastatic colorectal cancer", LANCET ONCOLOGY, vol. 12, 15 December 2010 (2010-12-15), pages 594-603, XP055180339, DOI: 10.1016/S1470-2045(10)70209-6**

- R. B. CORCORAN ET AL: "EGFR-Mediated Reactivation of MAPK Signaling Contributes to Insensitivity of BRAF-Mutant Colorectal Cancers to RAF Inhibition with Vemurafenib", CANCER DISCOVERY, vol. 2, no. 3, 16 January 2012 (2012-01-16), pages 227-235, XP055145702, ISSN: 2159-8274, DOI: 10.1158/2159-8290.CD-11-0341
- JING GAO ET AL: "Wild-type KRAS and BRAF could predict response to Cetuximab in Chinese colorectal cancer patients", CHINESE JOURNAL OF CANCER RESEARCH, CHINESE ANTI-CANCER ASSOCIATION, HEIDELBERG, vol. 23, no. 4, 22 December 2011 (2011-12-22), pages 271-275, XP019991201, ISSN: 1993-0631, DOI: 10.1007/S11670-011-0271-4
- RAZZAQUE M ABDUR ET AL: "Germline gain-of-function mutations in RAF1 cause Noonan syndrome", NATURE GENETICS, NATURE PUBLISHING GROUP, NEW YORK, US, vol. 39, no. 8, 1 August 2007 (2007-08-01) , pages 1013-1017, XP002564396, ISSN: 1061-4036, DOI: 10.1038/NG2078 [retrieved on 2007-07-01]
- KAZUKO SAKAI ET AL: "Extended RAS and BRAF Mutation Analysis Using Next-Generation Sequencing", PLOS ONE, vol. 10, no. 5, 8 May 2015 (2015-05-08), page e0121891, XP055622120, DOI: 10.1371/journal.pone.0121891
- SHIMADA YOSHIFUMI ET AL: "Clinical Significance of BRAF Non-V600E Mutations in Colorectal Cancer: A Retrospective Study of Two Institutions", JOURNAL OF SURGICAL RESEARCH, vol. 232, December 2018 (2018-12), pages 72-81, XP002794318,
- DI NICOLANTONIO, F. et al.: "Wild-type BRAF is required for response to panitumumab or cetuximab in metastatic colorectal cancer", Journal of Clinical Oncology, vol. 26, no. 35, 2008, pages 5705-5712, XP009123180,
- TRAN, B. et al.: "Impact of BRAF mutation and microsatellite instability on the pattern of metastatic spread and prognosis in metastatic colorectal cancer", Cancer, vol. 117, no. 20, 2011, pages 4623-4632, XP055435187,
- KALENDER ATAK, Z. et al.: "High accuracy mutation detection in leukemia on a selected panel of cancer genes", PLoS One, vol. 7, no. 6, 2012, page e38463, XP055435434,
- TISSOT, C. et al.: "Clinical characteristics and outcome of patients with lung cancer harboring BRAF mutations", Lung Cancer, vol. 9, no. 1, January 2016 (2016-01), pages 23-28, XP029368907,
- YOSUKE TAJIMA et al.: "A Systematic Analysis of Oncogene and Tumor Suppressor Genes for Panitumumab-Resistant Rectal Cancer with Wild RAS Gene -A Case Report", Japanese Journal of Cancer and Chemotherapy = Gan-to-kagaku-ryoho, vol. 43, no. 12, November 2016 (2016-11), pages 2280-2282, XP009513140, Tokyo ISSN: 0385-0684

## Description

### Technical Field

The present invention relates to a method for determining tolerance of a cancer cell to an epidermal growth factor receptor inhibitor (hereinafter will be referred to as "EGFR inhibitor") in a human patient suffering from a cancer.

### Background Art

EGFR inhibitors, for example, anti-epidermal growth factor receptor antibody drugs (anti-EGFR antibody drug) such as cetuximab and panitumumab have been known as a therapeutic agent for cancer. These EGFR inhibitors act in cancer cells to inhibit the function of an epidermal growth factor receptor (EGFR) which is involved in cancer cell growth.
Meanwhile, it has been known that, in a case where cancer cells of a cancer patient have a mutation in the KRAS gene, the cancer cells are resistant to the EGFR inhibitor, and therefore effects of the EGFR inhibitor are reduced. The EGFR inhibitor has side effects such as skin disorder, and therefore it is preferable to administer the EGFR inhibitor only to cancer patients for whom high therapeutic efficacy may be expected by administration of the EGFR inhibitor. As a method for determining whether a treatment with the EGFR inhibitor is effective or not to the patient, a method for checking the presence or absence of a mutation in the KRAS gene of the cancer cells of the patient has been known.
For example, Patent Literature 1 discloses a method for predicting sensitivity to EGFR inhibitors, in which, in a case where a nucleic acid derived from a mutant-type KRAS gene or a protein thereof is detected in a blood sample, it is determined that a possibility that a tumor is not sensitive to the EGFR inhibitor is high.
According to de Roock et al, Lancet Oncology, vol. 12, 2011, p. 594 - 603, BRAF mutation V600E confers resistance of cancer patients to EGFR antibodies.

### Citation List

#### Patent Literature

[Patent Literature 1] PCT International Publication No. 2014/148557

### Summary of Invention

#### Technical Problem

The inventors of the present invention have found that the treatment with the EGFR inhibitor is not effective in some cases even to the cancer patient whose cancer cells have the wild-type KRAS gene.
The present invention has been made in view of the above problems, and an object of the present invention is to select in advance a cancer patient for whom a treatment with the EGFR inhibitor is ineffective.

### Solution to Problem

The present invention is a method for determining tolerance of a cancer cell to an EGFR inhibitor in a human patient suffering from a cancer, the method comprising a step of determining the presence or absence of a mutation of a 326th amino acid residue of an amino acid sequence of a B-Raf protein (hereinafter referred to as "B-Raf') of the cancer cell by using a sample containing the cancer cell collected from the human patient, wherein the cancer cell is determined as tolerant to the epidermal growth factor receptor inhibitor, when the mutation of the amino acid residue is present.
According to this method, it is possible to determine the tolerance of the cancer cell to the EGFR inhibitor by determining the presence or absence of the mutation of a specific amino acid residue, which is the 326th amino acid residue, in the amino acid sequence of the B-Raf of the cancer cell, and therefore it is possible to select in advance a cancer patient for whom a treatment with the EGFR inhibitor is ineffective.
The above-described EGFR inhibitor may be an anti-epidermal growth factor receptor antibody drug.
The determination of the presence or absence of the mutation of the amino acid residue described above may include detection of a mutation of a base sequence encoding the 326th amino acid residue of the amino acid sequence of the B-Raf.
The detection of the mutation of the base sequence described above may be performed by DNA sequencing, polymerase chain reaction, allele-specific amplification, hybridization using allele-specific probes, mismatch cleavage analysis, single-strand conformation polymorphism, denaturing gradient gel electrophoresis, or temperature gradient gel electrophoresis.

The above-described sample may be a cancer resection tissue specimen, a biopsy specimen, an ascites-infiltrating cancer cell, a circulating cancer cell, serum, or plasma. If these samples are used as a sample, it is possible to select with higher certainty a cancer patient for whom the treatment with the EGFR inhibitor is ineffective.

The above-described cancer may be a colorectal cancer or a rectal cancer. If the cancer is a colorectal cancer or a rectal cancer, it is possible to select with higher certainty a cancer patient for whom the treatment with the EGFR inhibitor is ineffective.

The mutation of the amino acid residue described above may be I326V. If the mutation is I326V, it is possible to select with higher certainty a cancer patient for whom the treatment with the EGFR inhibitor is ineffective.

The mutation of the base sequence described above may be c.976A>G. If the mutation is c.976A>G, it is possible to select with higher certainty a cancer patient for whom the treatment with the EGFR inhibitor is ineffective.

The above-described cancer cell preferably has a wild-type KRAS gene. With the cancer cell having the wild-type KRAS gene, it is possible to select with higher certainty a cancer patient for whom the treatment with the EGFR inhibitor is ineffective.

**Advantageous Effects of Invention**

According to the present invention, it is possible to select in advance a cancer patient for whom the treatment with the EGFR inhibitor is ineffective.

**Brief Description of Drawings**

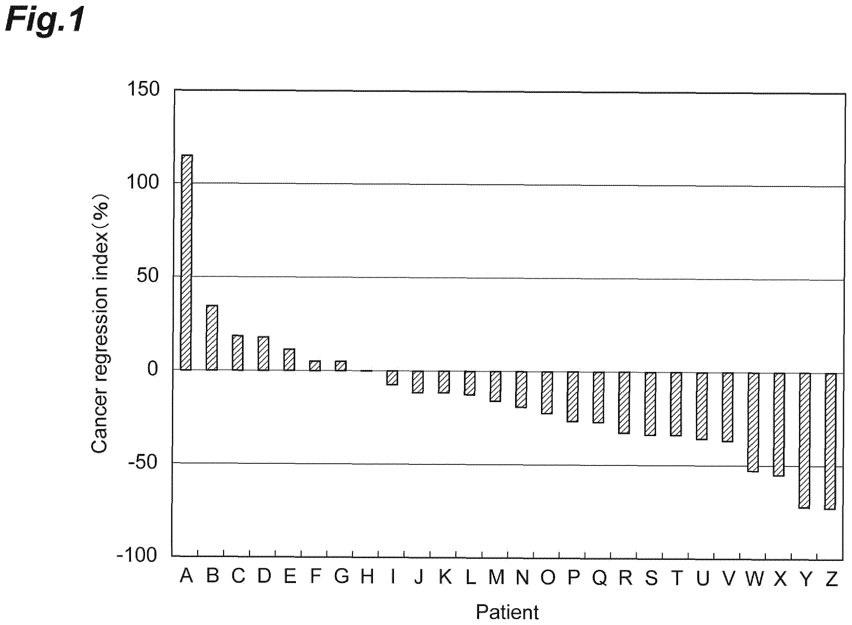

Fig. 1 is a graph showing results of an example.

**Description of Embodiments**

Hereinafter, embodiments for carrying out the present invention will be described in detail. However, the present invention is not limited to the following embodiments.

In one aspect, the present invention is a method for determining tolerance of a cancer cell to an EGFR inhibitor in a human patient suffering from a cancer (hereinafter referred to as "cancer patient"). The method according to this aspect comprises a step of determining the presence or absence of a mutation of a 326th amino acid residue of an amino acid sequence of a B-Raf of the cancer cell by using a sample containing the cancer cell collected from the human patient.

Examples of cancers for which the method according to the present invention may be used include colorectal cancer, rectal cancer, colon cancer, stomach cancer, liver cancer, thyroid cancer, uterine cancer, kidney cancer, pancreatic cancer, tongue cancer, prostate cancer, lung cancer, skin cancer, ovarian cancer, gallbladder cancer, head and neck cancer, testicular cancer, adrenal cancer, oral cancer, bone and soft tissue tumor, brain tumor, malignant melanoma, osteosarcoma, chondrosarcoma, rhabdomyosarcoma, leiomyosarcoma, leukemia, malignant lymphoma, and multiple myeloma. Among these, in a case where the cancer is a colorectal cancer or a rectal cancer, it is possible to select with higher certainty a cancer patient for whom a treatment with the EGFR inhibitor is ineffective.

The "EGFR inhibitor" in the present description is not particularly limited as long as the EGFR inhibitor is a drug that inhibits expression or activity of the EGFR, and may be any of a low molecular compound targeting the EGFR, such as gefitinib and erlotinib, an anti-EGFR antibody drug, an EGFR antisense oligonucleotide, an aptamer, and the like. The anti-EGFR antibody drug is, for example, an antibody that inhibits binding of epidermal growth factor (EGF) to the EGFR. Examples of the anti-EGFR antibody drug include a monoclonal antibody that recognizes an extracellular domain of the EGFR as an epitope. Specific examples of the anti-EGFR antibody drug include cetuximab and panitumumab. The EGFR inhibitor may be used alone, or a plurality of EGFR inhibitors may be used in combination.

The "antibody" in the present description encompasses not only an antibody molecule having two complete light chains and two complete heavy chains, but also an antibody fragment capable of binding to an antigen. Examples of the antibody fragment include F(ab')2, Fab', Fab, and Fv. The antibody is preferably either a chimeric antibody, a humanized antibody, or a fully human antibody.

In addition, the method according to the present embodiment is effective also in a case of determining the tolerance of the cancer cell to a treatment in which the EGFR inhibitor and other anticancer agent are used in combination. Examples of the treatment in which the EGFR inhibitor and other anticancer agent are used in combination include CPT-11 + Panitumumab therapy, IRIS + Panitumumab therapy, FOLFOX (for example, mFOLFOX6) + Panitumumab therapy, FOLFIRI + Panitumumab therapy, CPT-11 + Cetuximab therapy, IRIS + Cetuximab therapy, FOLFOX (for example, mFOLFOX6) + Cetuximab therapy, FOLFIRI + Cetuximab therapy, and sLV5FU2 + Cetuximab therapy.

Examples of the sample containing the cancer cell include a cancer resection tissue specimen, a biopsy specimen, an ascites-infiltrating cancer cell, a circulating cancer cell, serum, plasma, blood, feces, urine, sputum, cerebrospinal fluid, pleural fluid, nipple aspirate fluid, lymph fluid, cell culture liquid, and other tissues and fluids collected from the patient. From the viewpoint of selecting with higher certainty a cancer patient for whom a treatment with the EGFR inhibitor is ineffective, the sample containing the cancer cell is preferably a cancer resection tissue specimen, a biopsy specimen, an ascites-infiltrating cancer cell, a circulating cancer cell, serum, or plasma, and more preferably a cancer resection tissue specimen or a biopsy specimen. In addition, in a case where the sample containing the cancer cell is the cancer resection tissue specimen or the biopsy specimen, these specimens may be subjected to freezing, alcohol fixation, formalin fixation, paraffin wrapping, or a combination of these treatments.

In the present description, "mutation of the amino acid residue" means that a specific amino acid residue in an amino acid sequence of a protein is substituted with an amino acid residue different from an amino acid residue in a corresponding wild-type amino acid sequence. For example, the 326th amino acid residue of the amino acid sequence of the wild-type B-Raf shown in SEQ ID NO: 1 is isoleucine, and substitution of this amino acid residue with an amino acid residue other than isoleucine is called the mutation.

The mutation of the 326th amino acid residue of the amino acid sequence of the B-Raf may be a mutation in which the isoleucine is substituted with phenylalanine, threonine, aspartic acid, lysine, serine, arginine, methionine, glycine, alanine, valine, or leucine. In a case where the mutation of the amino acid residue is the mutation described above, it is possible to select with higher certainty a cancer patient for whom a treatment with the EGFR inhibitor is ineffective. Among these, in a case where the mutation is a mutation (I326V) in which the isoleucine has been substituted with valine, it is possible to select with higher certainty a cancer patient for whom a treatment with the EGFR inhibitor is ineffective.

The determination of the presence or absence of the mutation of the amino acid residue described above may be performed by known methods. The determination of the presence or absence of the mutation may include, for example, detection of a mutation of a base sequence that encodes the 326th amino acid residue of the amino acid sequence of the B-Raf.

In the present description, "mutation of the base sequence" means that at least a part of the bases in the base sequence is substituted with other base such that the amino acid residue encoded by the base sequence becomes different from an amino acid residue encoded by a corresponding wild-type base sequence (also called as a "missense mutation").

The mutation of the base sequence that encodes the 326th amino acid residue of the amino acid sequence of the B-Raf may be a mutation in which the amino acid residue encoded by the base sequence is altered from isoleucine to phenylalanine, threonine, aspartic acid, lysine, serine, arginine, methionine, glycine, alanine, valine, or leucine. In a case where the mutation of the base sequence is the mutation described above, it is possible to select with higher certainty a cancer patient for whom the treatment with the EGFR inhibitor is ineffective. Among these, in a case where the mutation of the base sequence is a mutation in which the base sequence encoding the isoleucine is mutated to a base sequence encoding valine (c.976A>G), it is possible to select with higher certainty a cancer patient for whom the treatment with the EGFR inhibitor is ineffective.

Detection of the mutation of the base sequence may be performed by known methods. The detection of the mutation of the base sequence may be performed by, for example, DNA sequencing, polymerase chain reaction, allele-specific amplification, hybridization using allele-specific probes, mismatch cleavage analysis, single-strand conformation polymorphism, denaturing gradient gel electrophoresis, or temperature gradient gel electrophoresis. The technique may be used alone, or a plurality of techniques may be used in combination.

When the mutation exists on the 326th amino acid residue of the amino acid sequence of the B-Raf of the cancer cell, it is possible to determine that the cancer cell is tolerant to the EGFR inhibitor.

The mechanism between the presence or absence of the mutation of the 326th amino acid residue of the amino acid sequence of the B-Raf of the cancer cell and the tolerance of the cancer cell to the EGFR inhibitor is not certain; however, the inventors of the present invention speculate that the presence or absence of the mutation, and the tolerance of the cancer cell are related to each other at least in the following mechanism. The B-Raf is an activation factor present downstream of the EGFR in an intracellular growth signaling pathway involved in cancer cell growth. The EGFR inhibitor has an effect in which growth signals are inhibited from being transmitted to downstream of the EGFR by inhibiting the function of EGFR, and thus the progression of cancer is suppressed. However, if the mutation exists on the 326th amino acid residue of the amino acid sequence of the B-Raf of the cancer cell, the B-Raf is activated even when the growth signals are not transmitted to downstream of the EGFR due to the EGFR inhibitor, and thus the growth signals are transmitted to downstream of the B-Raf. Therefore, it is considered that, even if the EGFR inhibitor is administered to such a cancer cell, the effect of suppressing the progression of cancer is not exerted, or is unlikely to be exerted.

The present invention may also said to be a method for determining tolerance of the cancer cell to the EGFR inhibitor in a human patient suffering from a cancer, the method including a step of determining the presence or absence of the mutation of the 326th amino acid residue of the amino acid sequence of the B-Raf of the cancer cell by using the sample containing the cancer cell collected from the human patient, wherein the presence of the mutation of the amino acid residue indicate that the cancer cell is tolerant to the anti-EGR antibody drug.

In one embodiment, the cancer cell collected from the cancer patient may have the wild-type KRAS gene. The treatment with the EGFR inhibitor is not effective in some cases even if the cancer cells of the cancer patient have the wild-type KRAS gene. According to this embodiment, by evaluating the efficacy of the EGFR inhibitor in the cancer patient whose cancer cells have the wild-type KRAS gene, it is possible to avoid unnecessary side effects caused by the EGFR inhibitor. In the present description, a wild-type KRAS gene means a KRAS gene having no mutation that gives cancer cells tolerance to the EGFR inhibitor. The mutation is a gene mutation that causes an alteration in the type of 12th and 13th amino acids of the KRAS protein.

In one embodiment, the cancer patient may a patient who intends to undergo or has undergone a treatment with medication with the EGFR inhibitor. According to this embodiment, it is possible to avoid performing the treatment with medication with the EGFR inhibitor in a patient having cancer cells tolerant to the EGFR inhibitor, or to reduce a dose of the EGFR inhibitor. Accordingly, the side effects caused by the EGFR inhibitor can be avoided or reduced.

In one embodiment, the cancer cell collected from the cancer patient may have a wild-type NRAS gene as well as a wild-type KRAS gene. Generally, a treatment with the EGFR inhibitor is considered to be effective for the cancer patients whose cancer cells have a wild-type NRAS gene as well as wild-type KRAS gene. However, even among such patients, there are patients for whom the treatment with the EGFR inhibitor is ineffective. According to this embodiment, it is possible to discriminate the patients for whom the treatment with the EGFR inhibitor is ineffective among such cancer patients. In the present description, a wild-type NRAS gene means a NRAS gene having no mutation that gives cancer cells tolerance to the EGFR inhibitor. The mutation, for example, is a gene mutation that causes an alteration in the type of a 12th, 13th, 59th, 61st, 117th, or 146th amino acid of the NRAS protein.

In one aspect, the present invention is a method for determining prognosis of cancer, that is a method which involves determining the presence or absence of the mutation of the 326th amino acid residue of the amino acid sequence of the B-Raf of the cancer cell by using the sample containing the cancer cell collected from the human patient suffering from the cancer, wherein the prognosis is determined as poor, when the mutation of the amino acid residue is present.

Hereinbefore, the specific embodiments of the present invention were described in detail, but the present invention is not limited to the above-described embodiments.

Examples

Hereinafter, the present invention will be more specifically described based on examples, but the present invention is not limited to the following examples.

(Detection of Mutation in BRAF Gene)

Patients with a colorectal cancer not having a mutation in codons of the 12th and 13th amino acids of the KRAS protein were treated with a therapy using Panitumumab or Cetuximab (shown in Table 1). A formalin-fixed and paraffin-embedded specimen was prepared from a cancer resection specimen of the patient, and cut into thin sections to prepare two sections with a thickness of 20 $\mu$m. A sample obtained by attaching the prepared two sections to a slide glass was used as a sample for DNA extraction. Separately, a sample obtained by preparing a section with a thickness of 4 $\mu$m, and attaching the prepared section to a slide glass was used as a sample for microscopic observation. Samples of 26 patients (patients A to Z) were prepared, and mutations in the base sequence of the BRAF gene were detected for each patient sample as follows.

[Table 1]

| Patient | Therapy | B-Raf mutation | Cancer regression index (%) |
|---|---|---|---|
| A | IRIS + Panitumumab | 1326V | 115 |
| B | IRIS + Panitumumab | - | 34.3 |
| C | IRIS + Panitumumab | - | 18.5 |
| D | IRIS + Panitumumab | - | 17.8 |
| E | IRIS + Panitumumab | - | 11.3 |
| F | IRIS + Panitumumab | D22N | 5 |
| G | FOLFIRI + Cetuximab | - | 5.1 |

6

(continued)

| Patient | Therapy | B-Raf mutation | Cancer regression index (%) |
|---|---|---|---|
| H | IRIS + Panitumumab CPT - 11 + Cetuximab | | 0.1 |
| I | IRIS + Panitumumab | - | -7.4 |
| J | CPT - 11 + Cetuximab | - | -11.6 |
| K | mFOLFOX6 + Panitumumab | V600E | -11.5 |
| L | IRIS + Panitumumab | - | -12.5 |
| M | mFOLFOX6 + Cetuximab | - | -15.9 |
| N | IRIS + Panitumumab | - | -19.1 |
| O | IRIS + Panitumumab mFOLFOX6 + Cetuximab sLV5FU2 + Cetuximab | N581Y | -22.4 |
| P | IRIS + Panitumumab | - | -27 |
| Q | FOLFIRI + Cetuximab | - | -26.6 |
| R | CPT - 11 + Cetuximab | - | -32.8 |
| S | mFOLFOX6 + Panitumumab | - | - -33.8 |
| T | IRIS + Panitumumab | - | -33.7 |
| U | CPT - 11 + Panitumumab | V600E | -35.7 |
| V | CPT - 11 + Panitumumab | - | - -36.8 |
| W | IRIS + Panitumumab | - | - -52.8 |
| X | mFOLFOX6 + Cetuximab | - | - -55 |
| Y | mFOLFOX6 + Panitumumab | - | -72.3 |
| Z | IRIS + Panitumumab | - | -72.7 |

The sample for microscopic observation was stained with hematoxylin and eosin. The sample after the staining was observed with a microscope and a site containing many cancer cells in the section was specified. From the sample for DNA extraction, the site specified in the sample for microscopic observation was scraped with a razor, and DNA was extracted from the scraped site. The DNA extraction was carried out by using BiOstic FFPE Tissue DNA Isolation Kit (trade name).

The BRAF gene was isolated from the DNA of the specimen by using, as a probe, a probe nucleic acid containing a part or all of continuous sequences (100 to 130 bases) in each of 18 exon sequences of the BRAF gene, or containing a complementary sequences thereof. The base sequence of the BRAF gene was analyzed by MiSeq sequencer of Illumina, Inc. to detect mutations in the base sequence of the BRAF gene.

For the patient A, the mutation (c.976A>G) that causes the mutation of I326V in the amino acid sequence of the B-Raf was detected in the base sequence of the BRAF gene. For the patients F, K, O, and U, mutations that cause mutations of D22N, V600E, N581Y, and V600E, respectively, were detected in the amino acid sequence of the B-Raf in the base sequence of the BRAF gene. For the other patients, no mutation that causes a mutation in the amino acid sequence of the B-Raf was detected in the base sequence of the BRAF gene.

(Effect of Treatment with Anti-EGFR Antibody Drug)

For the patients A to Z, a cancer regression index (%) was calculated as the following formula.

$$\text{Cancer regression index (\%)} = (\text{diameter of primary lesion after treatment} + \text{diameter of metastatic lesion after treatment})/(\text{diameter of primary lesion before treatment} + \text{diameter of metastatic lesion before treatment}) \times 100 - 100$$

The relationship between the presence or absence of the mutation of the BRAF gene, and the effect of the treatment with the anti-EGFR antibody drug in the patients A to Z is shown in Fig. 1 and Tables 1 and 2.

[Table 2]

| B-Raf mutation | Number of patients | Average cancer regression index (%) | Standard deviation |
|---|---|---|---|
| 1326V | 1 | 115 | - |
| D22N V600E N581 Y | 4 | -16.3 | 17.3 |
| No mutation | 22 | -19 | 28.9 |

As shown in Fig. 1 and Tables 1 and 2, in the patient A for whom the mutation that causes the mutation in the 326th amino acid residue of the amino acid sequence of the B-Raf was detected in the base sequence of the BRAF gene, the progression of cancer was recognized, and the cancer cells of the patient A had tolerance to the anti-EGFR antibody drug.

**Claims**

1.  A method for determining tolerance of a cancer cell to an epidermal growth factor receptor inhibitor in a human patient suffering from a cancer, the method comprising:

    a step of determining the presence or absence of a mutation of a 326th amino acid residue of an amino acid sequence of a B-Raf protein of the cancer cell by using a sample containing the cancer cell collected from the human patient,
    wherein the cancer cell is determined as tolerant to the epidermal growth factor receptor inhibitor, when the mutation of the amino acid residue is present.

2.  The method according to claim 1,
    wherein the epidermal growth factor receptor inhibitor is an anti-epidermal growth factor receptor antibody drug.

3.  The method according to claim 1 or 2,
    wherein the determination of the presence or absence of the mutation of the amino acid residue includes detection of a mutation of a base sequence encoding the 326th amino acid residue of the amino acid sequence of the B-Raf protein.

4.  The method according to claim 3,
    wherein the detection of the mutation of the base sequence is performed by DNA sequencing, polymerase chain reaction, allele-specific amplification, hybridization using allele-specific probes, mismatch cleavage analysis, single-strand conformation polymorphism, denaturing gradient gel electrophoresis, or temperature gradient gel electrophoresis.

5.  The method according to any one of claims 1 to 4,
    wherein the sample is a cancer resection tissue specimen, a biopsy specimen, an ascites-infiltrating cancer cell, a circulating cancer cell, serum, or plasma.

6.  The method according to any one of claims 1 to 5,
    wherein the cancer is a colorectal cancer or a rectal cancer.

7.  The method according to any one of claims 1 to 6,
    wherein the mutation of the amino acid residue is I326V.

**8.** The method according to claim 3 or 4,
wherein the mutation of the base sequence is c.976A>G.

**9.** The method according to any one of claims 1 to 8,
wherein the cancer cell has a wild-type KRAS gene.

## Patentansprüche

**1.** Verfahren für die Bestimmung der Toleranz einer Krebszelle gegenüber einem Hemmer vom epidermalen Wachstumsfaktor-Rezeptor bei einem menschlichen Patienten, der an einem Krebs leidet, wobei das Verfahren Folgendes umfasst:

einen Schritt der Bestimmung der Gegenwart oder Abwesenheit einer Mutation eines 326. Aminosäurerests einer Aminosäuresequenz eines B-Raf-Proteins der Krebszelle durch Verwendung einer Probe, die die Krebszelle enthält, die von dem menschlichen Patienten entnommen wurde,
wobei die Krebszelle als tolerant gegenüber dem Hemmer vom epidermalen Wachstumsfaktor-Rezeptor bestimmt wird, wenn die Mutation des Aminosäurerests vorliegt.

**2.** Verfahren nach Anspruch 1,
wobei der Hemmer vom epidermalen Wachstumsfaktor-Rezeptor ein Antikörperarzneimittel gegen den epidermalen Wachstumsfaktor-Rezeptor ist.

**3.** Verfahren nach Anspruch 1 oder 2,
wobei die Bestimmung der Gegenwart oder Abwesenheit der Mutation des Aminosäurerests den Nachweis einer Mutation einer Basensequenz einschließt, die den 326. Aminosäurerest der Aminosäuresequenz des B-Raf-Proteins kodiert.

**4.** Verfahren nach Anspruch 3,
wobei der Nachweis der Mutation der Basensequenz durch Folgendes durchgeführt wird: DNA-Sequenzierung, Polymerasekettenreaktion, allelspezifische Amplifikation, Hybridisierung unter Verwendung von allelspezifischen Sonden, Mismatch-Spaltanalyse, Einzelstrang-Konformations-Polymorphismus, denaturierende Gradienten-Gelelektrophorese oder Temperaturgradientengelelektrophorese.

**5.** Verfahren nach einem der Ansprüche 1 bis 4,
wobei die Probe eine Krebsresektions-Gewebeprobe, eine Biopsieprobe, eine Aszites-infiltrierende Krebszelle, eine zirkulierende Krebszelle, Serum oder Plasma ist.

**6.** Verfahren nach einem der Ansprüche 1 bis 5,
wobei der Krebs ein Kolorektalkrebs oder ein Rektumkrebs ist.

**7.** Verfahren nach einem der Ansprüche 1 bis 6,
wobei die Mutation des Aminosäurerests I326V ist.

**8.** Verfahren nach Anspruch 3 oder 4,
wobei die Mutation der Basensequenz c.976A>G ist.

**9.** Verfahren nach einem der Ansprüche 1 bis 8,
wobei die Krebszelle ein Wildtyp-KRAS-Gen aufweist.

## Revendications

**1.** Méthode destinée à déterminer la tolérance d'une cellule cancéreuse vis-à-vis d'un inhibiteur du récepteur du facteur de croissance de l'épiderme, chez un patient humain souffrant d'un cancer, la méthode comprenant :

une étape consistant à déterminer la présence ou l'absence d'une mutation du 326ème résidu d'acide aminé d'une séquence d'acides aminés d'une protéine B-Raf de la cellule cancéreuse, en utilisant un échantillon contenant la cellule cancéreuse prélevée auprès du patient humain ;
où on détermine que la cellule cancéreuse est tolérante vis-à-vis de l'inhibiteur du récepteur du facteur de croissance de l'épiderme, lorsque la mutation du résidu d'acide aminé est présente.

2. Méthode selon la revendication 1, dans laquelle l'inhibiteur du récepteur du facteur de croissance de l'épiderme est un médicament de type anticorps anti-récepteur du facteur de croissance de l'épiderme.

3. Méthode selon la revendication 1 ou 2, dans laquelle la détermination de la présence ou de l'absence de la mutation du résidu d'acide aminé comporte la détection d'une mutation d'une séquence de base codant pour le 326ème résidu d'acide aminé de la séquence d'acides aminés de la protéine B-Raf.

4. Méthode selon la revendication 3, dans laquelle la détection de la mutation de la séquence de base est mise en œuvre par séquençage d'ADN, amplification en chaîne par polymérase, amplification spécifique d'allèles, hybridation à l'aide de sondes spécifiques d'allèles, analyse de clivages des mésappariements, polymorphisme de conformation des simples brins, électrophorèse sur gel en gradient dénaturant, ou électrophorèse sur gel à gradient de température.

5. Méthode selon l'une quelconque des revendications 1 à 4, dans laquelle l'échantillon est une pièce d'exérèse de tissu cancéreux, une pièce de biopsie, une cellule cancéreuse infiltrant l'ascite, une cellule cancéreuse circulante, du sérum, ou du plasma.

6. Méthode selon l'une quelconque des revendications 1 à 5, dans laquelle le cancer est un cancer colorectal ou un cancer rectal.

7. Méthode selon l'une quelconque des revendications 1 à 6, dans laquelle la mutation du résidu d'acide aminé est I326V.

8. Méthode selon la revendication 3 ou 4, dans laquelle la mutation de la séquence de base est c.976A>G.

9. Méthode selon l'une quelconque des revendications 1 à 8, dans laquelle la cellule cancéreuse possède un gène KRAS de type sauvage.

Fig.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014148557 PCT

**Non-patent literature cited in the description**

- **DE ROOCK et al.** *Lancet Oncology,* 2011, vol. 12, 594-603

SEQUENCE LISTING

<110> Denka Company Limited
Niigata University

<120> A method of determining resistance of cancer cell against anti-EGFR antibody drug

<130> FP17-0160-00

<160> 1

<170> PatentIn version 3.5

<210> 1
<211> 766
<212> PRT
<213> Homo sapiens

<400> 1

```
Met Ala Ala Leu Ser Gly Gly Gly Gly Gly Ala Glu Pro Gly Gln
1               5               10              15


Ala Leu Phe Asn Gly Asp Met Glu Pro Glu Ala Gly Ala Gly Ala Gly
            20              25              30


Ala Ala Ala Ser Ser Ala Ala Asp Pro Ala Ile Pro Glu Glu Val Trp
        35              40              45


Asn Ile Lys Gln Met Ile Lys Leu Thr Gln Glu His Ile Glu Ala Leu
        50              55              60


Leu Asp Lys Phe Gly Gly Glu His Asn Pro Pro Ser Ile Tyr Leu Glu
65              70              75              80


Ala Tyr Glu Glu Tyr Thr Ser Lys Leu Asp Ala Leu Gln Gln Arg Glu
                85              90              95


Gln Gln Leu Leu Glu Ser Leu Gly Asn Gly Thr Asp Phe Ser Val Ser
        100             105             110


Ser Ser Ala Ser Met Asp Thr Val Thr Ser Ser Ser Ser Ser Ser Leu
        115             120             125


Ser Val Leu Pro Ser Ser Leu Ser Val Phe Gln Asn Pro Thr Asp Val
        130             135             140


Ala Arg Ser Asn Pro Lys Ser Pro Gln Lys Pro Ile Val Arg Val Phe
145             150             155             160


Leu Pro Asn Lys Gln Arg Thr Val Val Pro Ala Arg Cys Gly Val Thr
                165             170             175
```

Val Arg Asp Ser Leu Lys Lys Ala Leu Met Met Arg Gly Leu Ile Pro
                180                     185                 190

Glu Cys Cys Ala Val Tyr Arg Ile Gln Asp Gly Glu Lys Lys Pro Ile
                195                     200                 205

Gly Trp Asp Thr Asp Ile Ser Trp Leu Thr Gly Glu Glu Leu His Val
                210                     215                 220

Glu Val Leu Glu Asn Val Pro Leu Thr Thr His Asn Phe Val Arg Lys
225                     230                     235                 240

Thr Phe Phe Thr Leu Ala Phe Cys Asp Phe Cys Arg Lys Leu Leu Phe
                    245                     250                 255

Gln Gly Phe Arg Cys Gln Thr Cys Gly Tyr Lys Phe His Gln Arg Cys
                260                     265                 270

Ser Thr Glu Val Pro Leu Met Cys Val Asn Tyr Asp Gln Leu Asp Leu
                275                     280                 285

Leu Phe Val Ser Lys Phe Phe Glu His His Pro Ile Pro Gln Glu Glu
                290                     295                 300

Ala Ser Leu Ala Glu Thr Ala Leu Thr Ser Gly Ser Ser Pro Ser Ala
305                     310                     315                 320

Pro Ala Ser Asp Ser Ile Gly Pro Gln Ile Leu Thr Ser Pro Ser Pro
                325                     330                 335

Ser Lys Ser Ile Pro Ile Pro Gln Pro Phe Arg Pro Ala Asp Glu Asp
                340                     345                 350

His Arg Asn Gln Phe Gly Gln Arg Asp Arg Ser Ser Ser Ala Pro Asn
                355                     360                 365

Val His Ile Asn Thr Ile Glu Pro Val Asn Ile Asp Asp Leu Ile Arg
                370                     375                 380

Asp Gln Gly Phe Arg Gly Asp Gly Gly Ser Thr Thr Gly Leu Ser Ala
385                     390                     395                 400

Thr Pro Pro Ala Ser Leu Pro Gly Ser Leu Thr Asn Val Lys Ala Leu
                    405                     410                 415

Gln Lys Ser Pro Gly Pro Gln Arg Glu Arg Lys Ser Ser Ser Ser Ser

```
                    420                      425                         430


        Glu Asp Arg Asn Arg Met Lys Thr Leu Gly Arg Arg Asp Ser Ser Asp
            435                 440                 445


        Asp Trp Glu Ile Pro Asp Gly Gln Ile Thr Val Gly Gln Arg Ile Gly
            450                 455                 460


        Ser Gly Ser Phe Gly Thr Val Tyr Lys Gly Lys Trp His Gly Asp Val
        465                 470                 475                 480


        Ala Val Lys Met Leu Asn Val Thr Ala Pro Thr Pro Gln Gln Leu Gln
                        485                 490                 495


        Ala Phe Lys Asn Glu Val Gly Val Leu Arg Lys Thr Arg His Val Asn
                    500                 505                 510


        Ile Leu Leu Phe Met Gly Tyr Ser Thr Lys Pro Gln Leu Ala Ile Val
                    515                 520                 525


        Thr Gln Trp Cys Glu Gly Ser Ser Leu Tyr His His Leu His Ile Ile
            530                 535                 540


        Glu Thr Lys Phe Glu Met Ile Lys Leu Ile Asp Ile Ala Arg Gln Thr
        545                 550                 555                 560


        Ala Gln Gly Met Asp Tyr Leu His Ala Lys Ser Ile Ile His Arg Asp
                        565                 570                 575


        Leu Lys Ser Asn Asn Ile Phe Leu His Glu Asp Leu Thr Val Lys Ile
                    580                 585                 590


        Gly Asp Phe Gly Leu Ala Thr Val Lys Ser Arg Trp Ser Gly Ser His
                    595                 600                 605


        Gln Phe Glu Gln Leu Ser Gly Ser Ile Leu Trp Met Ala Pro Glu Val
            610                 615                 620


        Ile Arg Met Gln Asp Lys Asn Pro Tyr Ser Phe Gln Ser Asp Val Tyr
        625                 630                 635                 640


        Ala Phe Gly Ile Val Leu Tyr Glu Leu Met Thr Gly Gln Leu Pro Tyr
                        645                 650                 655


        Ser Asn Ile Asn Asn Arg Asp Gln Ile Ile Phe Met Val Gly Arg Gly
                    660                 665                 670
```

```
Tyr Leu Ser Pro Asp Leu Ser Lys Val Arg Ser Asn Cys Pro Lys Ala
        675             680             685

Met Lys Arg Leu Met Ala Glu Cys Leu Lys Lys Lys Arg Asp Glu Arg
        690             695             700

Pro Leu Phe Pro Gln Ile Leu Ala Ser Ile Glu Leu Leu Ala Arg Ser
705             710             715             720

Leu Pro Lys Ile His Arg Ser Ala Ser Glu Pro Ser Leu Asn Arg Ala
            725             730             735

Gly Phe Gln Thr Glu Asp Phe Ser Leu Tyr Ala Cys Ala Ser Pro Lys
        740             745             750

Thr Pro Ile Gln Ala Gly Gly Tyr Gly Ala Phe Pro Val His
        755             760             765
```